(19) Europäisches Patentamt
European Patent Office
Office européen des brevets

(11) **EP 4 015 616 A1**

(12) **EUROPEAN PATENT APPLICATION**
published in accordance with Art. 153(4) EPC

(43) Date of publication:
**22.06.2022 Bulletin 2022/25**

(21) Application number: **20867457.2**

(22) Date of filing: **11.05.2020**

(51) International Patent Classification (IPC):
**C12M 1/00** (2006.01)        **C12M 1/34** (2006.01)
**G16B 40/00** (2019.01)

(52) Cooperative Patent Classification (CPC):
**C12M 1/00; C12M 1/34; G16B 40/00**

(86) International application number:
**PCT/JP2020/018808**

(87) International publication number:
**WO 2021/059577 (01.04.2021 Gazette 2021/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2019 JP 2019173364**

(71) Applicant: **FUJIFILM Corporation**
**Tokyo 106-8620 (JP)**

(72) Inventors:
• **NAKAMURA, Naoki**
**Ashigarakami-gun, Kanagawa 258-8577 (JP)**
• **HARAGUCHI, Nobuyuki**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **INFORMATION PROCESSING DEVICE, INFORMATION PROCESSING METHOD, AND INFORMATION PROCESSING PROGRAM**

(57)    An information processing apparatus acquires input information including at least one of a process condition, a culture medium component, or the number and diameters of cells in cell culture, and estimates a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

## FIG. 6

INPUT LAYER   INTERLAYER   OUTPUT LAYER

56

PROCESS CONDITION
CULTURE MEDIUM COMPONENT
NUMBER OF CELLS
DIAMETER OF CELLS

ANTIBODY QUALITY
CELL QUALITY

**Description**

**BACKGROUND OF THE INVENTION**

1. Field of the Invention

The present disclosure relates to an information processing apparatus, an information processing method, and an information processing program.

2. Description of the Related Art

JP2009-44974A discloses a method for constructing an estimation model for estimating a quality of cells. In the method, for two or more samples in which cells of the same species are cultured, images are acquired by capturing images of cells of each sample are captured at two or more time points with different culture times, and each acquired image is analyzed, thereby generating numerical data for two or more indicators of morphology of the cells. In the method, actual measurement data of the estimation target is provided for each sample, the generated numerical data is used as an input value, and the provided actual measurement data is used as a teacher value for fuzzy neural network analysis, thereby building an estimation model that indicates a combination of indicators effective for estimation that calculate an output value on the basis of the fuzzy rule.

**SUMMARY OF THE INVENTION**

Perfusion culture is a culture method of cells used in the production of biopharmaceutical drugs using antibodies produced from cells. Perfusion culture is a culture method in which a culture liquid containing cells is continuously filtered and discharged, while a fresh culture medium containing nutritional components is continuously supplied to a culture tank. Perfusion culture is also referred to as continuous culture.

In perfusion culture, parameters, which affect the quality of the antibody, such as process conditions, culture medium components, and the number of cells in cell culture, can be changed even during the cell culture process. Therefore, in perfusion culture, on the basis of the information on the cell culture at a certain time point, it is preferable to be able to estimate the quality of the antibody after elapse of a predetermined period from the certain time point. Specifically, for example, on the basis of the information at a certain time point, in a case where the quality of the antibody after elapse of the predetermined period is within an allowable range, the perfusion culture can be continued as it is, and in a case where the quality is out of the allowable range, the above-mentioned parameters can be adjusted. In such a case, it is possible to effectively support perfusion culture.

In the technique described in JP2009-44974A, the quality of cells is estimated from images obtained by capturing cells at two different time points using an estimation model. That is, the technique described in JP2009-44974A estimates the quality of cells from the degree of change in the image of cells at two different time points. Therefore, the technique described in JP2009-44974A cannot estimate the quality of the antibody from the information at a certain time point, and is therefore unable to effectively support the perfusion culture.

The present disclosure has been made in view of the above-mentioned circumstances, and provides an information processing apparatus, an information processing method, and an information processing program capable of effectively supporting perfusion culture.

The information processing apparatus of the present disclosure includes: an acquisition unit that acquires input information including at least one of a process condition, a culture medium component, or the number and diameters of cells in cell culture; and an estimation unit that estimates a quality of an antibody produced from the cells and a quality of the cells after elapse of a predetermined period from a time point at which the acquisition unit acquires the input information, on the basis of the input information and a trained model which is trained in advance using the input information, the quality of the antibody, and the quality of the cells.

The information processing apparatus of the present disclosure may further include an output unit that outputs a warning in a case where the quality of the antibody and the quality of the cells estimated by the estimation unit are out of an allowable range.

Further, in the information processing apparatus of the present disclosure, the output unit may further output at least one information included in the input information in which the quality of the antibody and the quality of the cells are within the allowable range.

Furthermore, in the information processing apparatus of the present disclosure, an input of the trained model may be a plurality of pieces of the input information acquired at a plurality of time points until the predetermined period as a period for cell proliferation has elapsed from start of the cell culture.

Moreover, the information processing method of the present disclosure causes a computer to execute: acquiring input information including at least one of a process condition, a culture medium component, or the number and diameters of cells in cell culture; and estimating a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

In addition, the information processing program of the present disclosure causes a computer to execute: acquiring input information including at least one of a process condition, a culture medium component, or the number and diameters of cells in cell culture; and estimating a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

According to the present disclosure, it is possible to effectively support perfusion culture.

## BRIEF DESCRIPTION OF THE DRAWINGS

Fig. 1 is a diagram showing an example of the configuration of a cell culture device according to each embodiment.
Fig. 2 is a block diagram showing an example of a hardware configuration of an information processing apparatus according to each embodiment.
Fig. 3 is a diagram showing an example of learning data according to a first embodiment.
Fig. 4 is a diagram for explaining the learning data according to the first embodiment.
Fig. 5 is a block diagram showing an example of a functional configuration in a learning phase of the information processing apparatus according to the first embodiment.
Fig. 6 is a diagram showing an example of a trained model according to the first embodiment.
Fig. 7 is a flowchart showing an example of learning processing according to the first embodiment.
Fig. 8 is a block diagram showing an example of a functional configuration in an operation phase of the information processing apparatus according to the first embodiment.
Fig. 9 is a diagram for explaining a level of contribution of an input of the trained model according to the first embodiment.
Fig. 10 is a diagram showing an example of a warning screen according to the first embodiment.
Fig. 11 is a flowchart showing an example of quality estimation processing according to the first embodiment.
Fig. 12 is a diagram for explaining a flow of development of an antibody drug.
Fig. 13 is a graph showing an example of transition in antibody quality in time series.
Fig. 14 is a diagram showing an example of learning data according to a second embodiment.
Fig. 15 is a diagram for explaining the learning data according to the second embodiment.
Fig. 16 is a block diagram showing an example of a functional configuration in a learning phase of the information processing apparatus according to the second embodiment.
Fig. 17 is a diagram showing an example of a trained model according to the second embodiment.
Fig. 18 is a flowchart showing an example of learning processing according to the second embodiment.
Fig. 19 is a block diagram showing an example of a functional configuration in an operation phase of the information processing apparatus according to the second embodiment.
Fig. 20 is a flowchart showing an example of quality estimation processing according to the second embodiment.

## DESCRIPTION OF THE PREFERRED EMBODIMENTS

Examples of embodiments for carrying out the technique of the present disclosure will be hereinafter described in detail with reference to the drawings.

[First Embodiment]

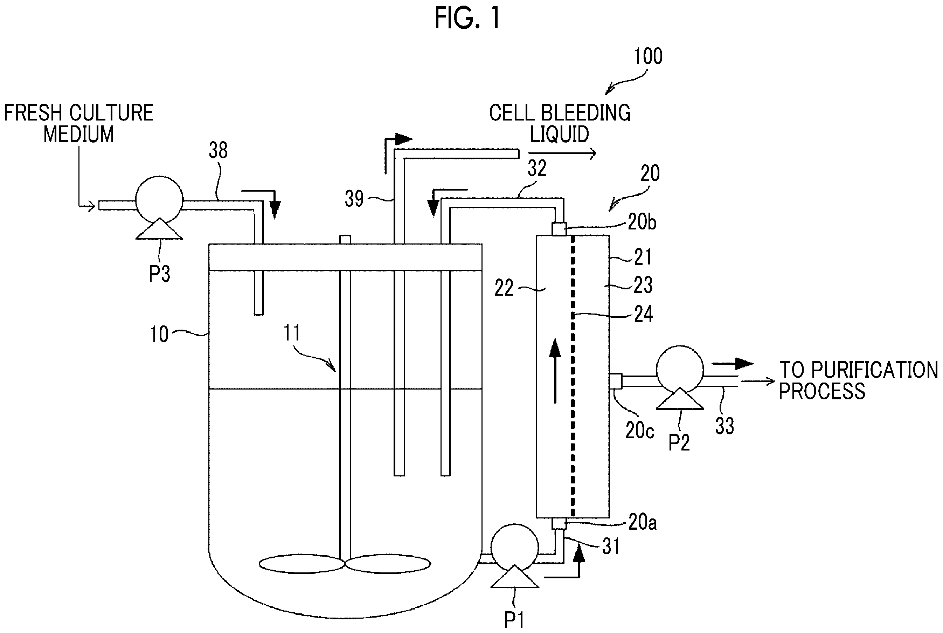

A configuration of a cell culture device 100 according to the present embodiment will be described with reference to Fig. 1. The cell culture device 100 can be suitably used in cell culture for expressing an antibody in animal cells, for example.

The cells used in expressing the antibody are not particularly limited, and examples thereof include animal cells, plant cells, eukaryotic cells such as yeast, prokaryotic cells such as grass bacillus, Escherichia coli, and the like. Animal cells such as CHO cells, BHK-21 cells, and SP2/0-Ag14 cells are preferable, and CHO cells are more preferable.

The antibody expressed in animal cells is not particularly limited, and includes, for example, anti-IL-6 receptor antibody, anti-IL-6 antibody, anti-glypican-3 antibody, anti-CD3 antibody, anti-CD20 antibody, anti-GPIIb/IIIa antibody, anti-TNF antibody, anti-CD25 antibody, anti-EGFR antibody, anti-Her2/neu antibody, anti-RSV antibody, anti-CD33 antibody, anti-CD52 antibody, anti-IgE antibody, anti-CD11a antibody, anti-VEGF antibody, anti-VLA4 antibody, and the like. The antibody includes not only monoclonal antibodies derived from animals such as humans, mice, rats, hamsters, rabbits, and monkeys, but also artificially modified antibodies such as chimeric antibodies, humanized antibodies, and bispecific antibodies.

The obtained antibody or fragment thereof can be purified to be uniform. For the separation and purification of the antibody or a fragment thereof, the separation and purification method used in a conventional polypeptide may be used. For example, an antibody can be separated and purified by appropriately selecting and combining a chromatography column such as affinity chromatography, a filter, ultrafiltration, salting out, dialysis, SDS polyacrylamide gel electrophoresis, and isoelectric point electrophoresis. However, the present invention is not limited thereto. The obtained concentration of the antibody can be measured by measurement of the absorbance or by an enzyme-linked immunosorbent assay (ELISA) or the like.

As shown in Fig. 1, the cell culture device 100 includes a culture container 10 that contains a cell suspension including cells, and a filter unit 20 that has a filter membrane 24 for subjecting the cell suspension extracted from the culture container 10 to a membrane separation treatment. The cell culture device 100 further includes a flow passage 32 as a circulation flow passage for returning components blocked by the filter membrane 24 to the culture container 10, and a flow passage 33 for discharging components having permeated through the filter membrane 24 of the cell suspension to the outside of the filter unit 20. Further, the cell culture device 100 includes a flow passage 38 for supplying a fresh culture medium to the culture container 10, and a pump P3 provided in the middle of the flow passage 38.

The culture container 10 is a container for containing a cell suspension including cells and a culture medium used in expressing an antibody. Inside the culture container 10, a stirring device 11 having a stirring blade is provided. By rotating the stirring blade of the stirring device 11, the culture medium contained together with the cells in the culture container 10 is stirred, and the homogeneity of the culture medium is maintained.

In the cell culture device 100, in order to prevent the concentration of cells in the culture container 10 from becoming excessively high, a cell bleeding treatment is performed, which is for bleeding off a part of the cells in the culture container 10 (for example, about 10%) at an appropriate timing during the culture period. In the cell bleeding treatment, the cells in the culture container 10 are discharged to the outside of the culture container 10 through the flow passage 39.

One end of the flow passage 31 is connected to the bottom of the culture container 10, and the other end is connected to an inlet 20a of the filter unit 20. In the middle of the flow passage 31, a pump P1, which extracts the cell suspension contained in the culture container 10 and sends the cell suspension to the filter unit 20, is provided.

The filter unit 20 comprises a container 21 and a filter membrane 24 that separates the space inside the container 21 into a supply side 22 and a permeation side 23 and performs a membrane separation treatment on the cell suspension extracted from the culture container 10. Further, the filter unit 20 has the inlet 20a through which the cell suspension flows in and an outlet 20b through which the cell suspension flows out on the supply side 22. The cell suspension extracted from the culture container 10 passes through the filter membrane 24 while flowing into the inside of the container 21 from the inlet 20a and flowing out to the outside of the container 21 from the outlet 20b. The filter unit 20 performs the membrane separation treatment by a tangential flow (cross flow) method of sending permeation components to the permeation side 23 while flowing a liquid subjected to the membrane separation treatment along the membrane surface of the filter membrane 24 subjected to the membrane separation treatment (that is, in a direction parallel to the membrane surface). The tangential flow method, which is a method for membrane separation treatment using the filter membrane 24, may be a method of forming a flow in which the cell suspension extracted from the culture container 10 circulates in one direction in parallel along the membrane surface of the filter membrane 24, or may be a method of forming a flow in which the cell suspension extracted from the culture container 10 reciprocates alternately in parallel along the membrane surface of the filter membrane 24. In a case of forming a circulating flow, for example, a KrosFlo perfusion culture flow path device (KML-100, KPS-200, and KPS-600) manufactured by Spectrum Laboratories Corp. can be suitably used. Further, in a case of forming a flow that reciprocates alternately, the ATF system manufactured by REPLIGEN Corp. can be suitably used.

The relatively large-sized components included in the cell suspension do not permeate through the filter membrane 24, flow out to the outside of the container 21 from the outlet 20b, and are returned to the inside of the culture container 10 through the flow passage 32. That is, in the cell suspension extracted from the culture container 10, the components blocked by the filter membrane 24 are returned to the inside of the culture container 10 through the flow passage 32. On the other hand, the relatively small-sized components included in the cell suspension permeate through the filter membrane 24 and are discharged to the outside of the container 21 from a discharge port 20c provided on the permeation side 23. A flow passage 33 provided with a pump P2 is connected to the discharge port 20c of the filter unit 20, and the components discharged to the permeation side 23 are discharged from the discharge port 20c to the outside of the container 21 through the flow passage 33.

In the cell culture device 100 according to the present embodiment, the filter membrane 24 is used for the purpose of separating cells and components unnecessary for cell culture. Examples of components unnecessary for cell culture include cell carcasses, cell crushed products, DNA, HCP, antibodies, waste products, and the like. That is, the filter membrane 24 has a separation performance suitable for blocking the permeation of cells while allowing components unnecessary for cell culture to permeate.

The components unnecessary for cell culture discharged from the culture container 10 as described above are sent to the next process, which is an antibody purification process.

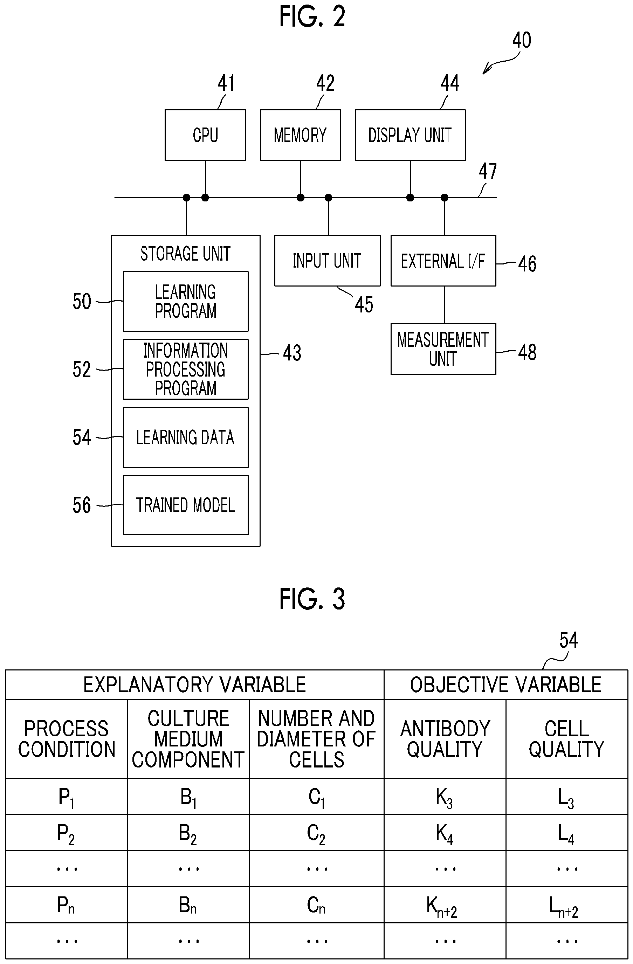

Next, referring to Fig. 2, a hardware configuration of an information processing apparatus 40 connected to the cell culture device 100 will be described. As shown in Fig. 2, the information processing apparatus 40 includes a central processing unit (CPU) 41, a memory 42, a storage unit 43, a display unit 44 such as a liquid crystal display, an input unit 45 such as a keyboard and a mouse, and an external interface (I/F) 46. The CPU 41, the memory 42, the storage unit 43, the display unit 44, the input unit 45, and the external I/F 46 are connected to the bus 47. The measurement unit 48 is connected to the external I/F 46. Examples of the information processing apparatus 40 include a personal computer, a server computer, and the like.

The storage unit 43 is realized by a hard disk drive (HDD), a solid state drive (SSD), a flash memory, or the like. A learning program 50 and an information processing program 52 are stored in the storage unit 43 as a storage medium. The CPU 41 reads the learning program 50 from the storage unit 43, expands the program into the memory 42, and executes the expanded learning program 50. Further, the CPU 41 reads the information processing program 52 from the storage unit 43, expands the program into the memory 42, and executes the expanded information processing program 52. Further, the learning data 54 and the trained model 56 are stored in the storage unit 43.

The measurement unit 48 includes various measurement devices each measuring the process conditions, the culture medium components, and the number and diameters of cells in cell culture using the cell culture device 100. Examples of process conditions include a rotation speed of the stirring device 11 per unit time (hereinafter referred to as "stirring rotation speed"), an aeration amount per unit volume of the culture medium contained in the culture container 10, and a temperature of the culture medium contained in the culture container 10. Further, examples of the culture medium components include an amount of nutritional component, an amount of metabolic component, an amount of dissolved gas (for example, an amount of dissolved oxygen), and the like of the culture medium contained in the culture container 10.

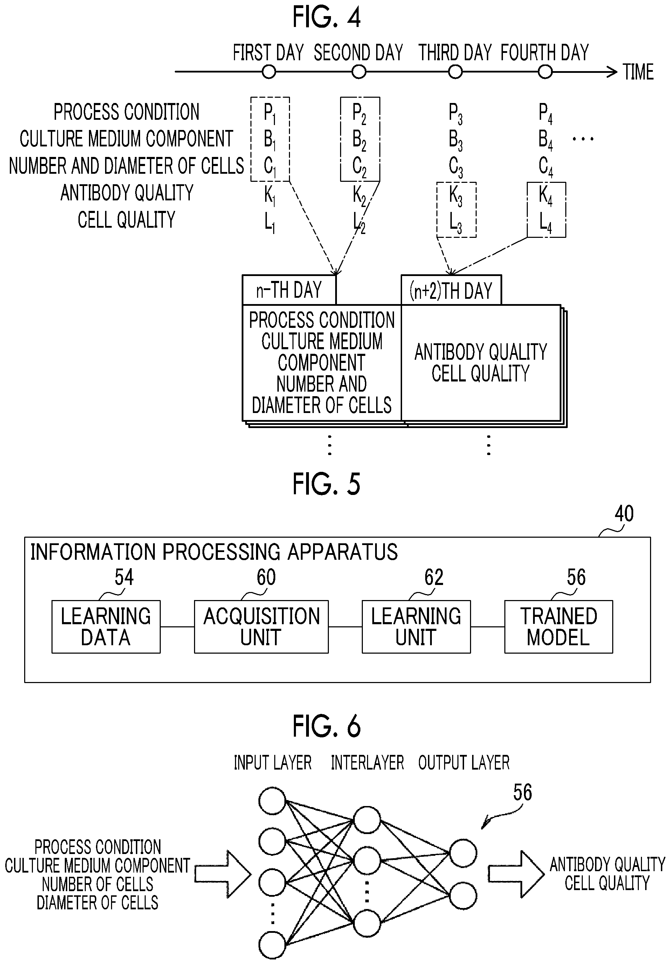

The details of the learning data 54 according to the present embodiment will be described with reference to Figs. 3 and 4. As shown in Fig. 3, the learning data 54 is a data set for learning that includes a plurality of sets of process conditions, culture medium components, and the number and diameters of cells in cell culture which are explanatory variables, a quality of the antibody produced from the cells which are objective variables corresponding to the explanatory variables, and a quality of the cells. The number of cells means a sum of the number of living cells and the number of dead cells. Examples of the quality of the antibody include a concentration of the antibody, an aggregate amount of the antibody, a decomposition product amount of the antibody, an immature sugar chain amount, and the like. Examples of quality of the cells include a cell survival rate and a cell viability. The quality of the antibody and the quality of the cells may be one of the index values or a combination of a plurality of index values. Further, the quality of the antibody and the quality of the cells may be evaluation values obtained by determining one or a plurality of combinations thereof in a plurality of stages (for example, four stages A to D) in accordance with a predetermined determination standard.

As shown in Fig. 4, each data included in the learning data 54 includes process conditions measured regularly (for example, once a day), culture medium components, and the number and diameters of cells, in the past cell culture. Each data included in the learning data 54 further includes the process conditions, the culture medium components, and the quality of the antibody and the quality of the cells after elapse of a predetermined period (for example, 2 days) from the measurement time point of the number and diameters of cells. The predetermined period is not limited to two days, and may be, for example, one day, three or more days, or a period other than one day (for example, 12 hours).

The trained model 56 is a model which is trained in advance using the learning data 54. An example of the trained model 56 is a neural network model. The trained model 56 is generated by the information processing apparatus 40 in the learning phase described later.

Next, referring to Fig. 5, a functional configuration in a learning phase of the information processing apparatus 40 will be described. As shown in Fig. 5, the information processing apparatus 40 includes an acquisition unit 60 and a learning unit 62. In a case where the CPU 41 executes the learning program 50, the CPU 41 functions as the acquisition unit 60 and the learning unit 62.

The acquisition unit 60 acquires the learning data 54 from the storage unit 43. The learning unit 62 generates the trained model 56 by training the model using the learning data 54 acquired by the acquisition unit 60 as training data. Then, the learning unit 62 stores the generated trained model 56 in the storage unit 43.

As an example, as shown in Fig. 6, by learning through the learning unit 62, the trained model 56 in which the process conditions, the culture medium components, the number and diameters of cells are input, and the quality of the antibody and the quality of cells are output are generated. For example, an error back propagation method is used in learning performed by the learning unit 62. The trained model 56 may be a deep neural network model having a plurality of interlayers.

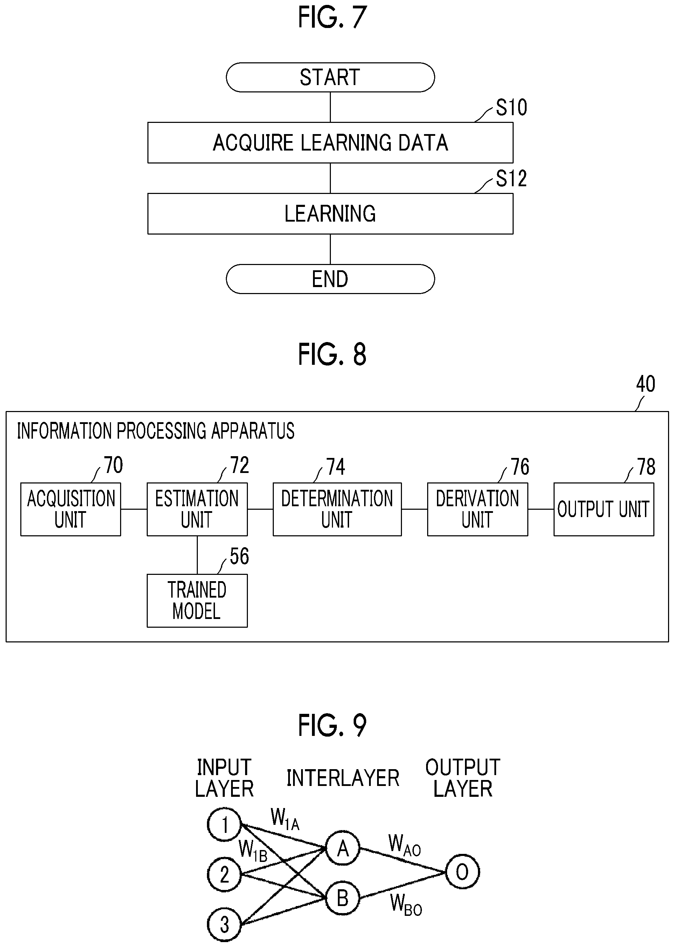

Next, referring to Fig. 7, an operation of the information processing apparatus 40 according to the present embodiment in the learning phase will be described. In a case where the CPU 41 executes the learning program 50, the learning processing shown in Fig. 7 is executed.

In step S10 of Fig. 7, the acquisition unit 60 acquires the learning data 54 from the storage unit 43. In step S12, as described above, the learning unit 62 generates the trained model 56 by training the model using the learning data 54 acquired in step S10 as the training data. Then, the learning unit 62 stores the generated trained model 56 in the storage unit 43. In a case where step S12 ends, the learning processing ends.

Next, referring to Fig. 8, a functional configuration in the operation phase of the information processing apparatus 40 according to the present embodiment will be described. As shown in Fig. 8, the information processing apparatus 40 includes an acquisition unit 70, an estimation unit 72, a determination unit 74, a derivation unit 76, and an output unit 78. In a case where the CPU 41 executes the information processing program 52, the CPU 41 functions as an acquisition unit 70, an estimation unit 72, a determination unit 74, a derivation unit 76, and an output unit 78.

The acquisition unit 70 acquires the process conditions, the culture medium components, and the number and diameters of cells in the cell culture using the cell culture device 100 measured by the measurement unit 48 at a predetermined periodic timing (for example, once a day). The process condition, the culture medium component, and the number and diameters of cells are examples of input information input to the trained model 56.

The estimation unit 72 estimates the quality of the antibody and the quality of the cells after elapse of a predetermined period (for example, 2 days) from the time point at which the acquisition unit 70 acquires the input information, on the basis of the input information acquired by the acquisition unit 70 and the trained model 56. Specifically, the estimation unit 72 inputs the input information, which is acquired by the acquisition unit 70, to the trained model 56. As described above, the trained model 56 is a model that is trained in a case of inputting the process conditions, the culture medium components, the number and diameters of cells and outputting the quality of the antibody and the quality of the cells after elapse of the predetermined period. Therefore, the output from the trained model 56 is estimated values of the quality of the antibody and the quality of the cells after the predetermined period has elapsed from the time point at which the acquisition unit 70 acquires the input information. As described above, the estimation unit 72 estimates the quality of the antibody and the quality of the cells after the predetermined period has elapsed from the time point at which the acquisition unit 70 acquires the input information.

The determination unit 74 determines whether the quality of the antibody and the quality of the cells estimated by the estimation unit 72 are within the allowable range or out of the allowable range. The allowable range is experimentally determined in advance in accordance with, for example, the cell type and the index value used as the quality of the antibody. For example, in a case where the aggregate amount of the antibody is used as the quality of the antibody, the determination unit 74 determines that the quality of the antibody is out of the allowable range in a case where the aggregate amount of the antibody is equal to or greater than a threshold value, and determines that the quality of the antibody is within the allowable range in a case where the aggregate amount of the antibody is less than the threshold value.

In a case where the determination unit 74 determines that the quality of the antibody and the quality of the cells are out of the allowable range, the derivation unit 76 derives one information piece of the information pieces included in the input information in which the quality of the antibody and the quality of the cells are within the allowable range. Specifically, the derivation unit 76 derives the level of contribution of each explanatory variable (that is, each input) of the trained model 56 to the objective variable (that is, the output), and derives the explanatory variable having the highest level of contribution, as one information piece in which the quality of the antibody and the quality of the cells are within the allowable range.

Referring to Fig. 9, the derivation processing of the level of contribution by the derivation unit 76 will be specifically described. Here, in order to facilitate understanding of the description, description will be given of an example in which the input layer of the trained model 56 has three nodes 1 to 3, the interlayer thereof has two nodes A and B, and the output layer thereof has one node O.

The derivation unit 76 derives the inner product of the weights as the level of contribution for each input node. In the example of Fig. 9, the derivation unit 76 derives the level of contribution of the node 1 in accordance with Expression (1). $W_{1A}$ in Expression (1) is a weight for nodes 1 to A, and $W_{AO}$ is a weight for nodes A to O. Further, $W_{1B}$ in Expression (1) is a weight for nodes 1 to B, and $W_{BO}$ is a weight for nodes B to O. It is possible to derive the level of contribution for the node 2 and the node 3 in a similar manner.

$$\text{Level of contribution of node } 1 = W_{1A} \times W_{AO} + W_{1B} \times W_{BO} \dots (1)$$

Further, the derivation unit 76 also derives change information indicating how the derived explanatory variable having the highest level of contribution is changed in accordance with whether the sign of the level of contribution is positive or negative. As a specific example, description will be given of a case where the explanatory variable having the highest level of contribution is a stirring rotation speed of the stirring device 11 included in the process conditions and the quality of the antibody is an aggregate amount of the antibody. In such a case, in a case where the sign of the level of contribution is a positive sign, the derivation unit 76 derives the information indicating that the stirring rotation speed is reduced as the above-mentioned change information. On the other hand, in a case where the sign of the level of contribution is a negative sign, the derivation unit 76 derives the information indicating that the stirring rotation speed is increased as the above-mentioned change information.

The derivation unit 76 may derive, for example, a plurality of explanatory variables in descending order of the level of contribution, instead of one explanatory variable having the highest level of contribution, as information that the quality of the antibody and the quality of the cells are within the allowable range, and may derive one or more explanatory variables of which the level of contribution is equal to or greater than the threshold value.

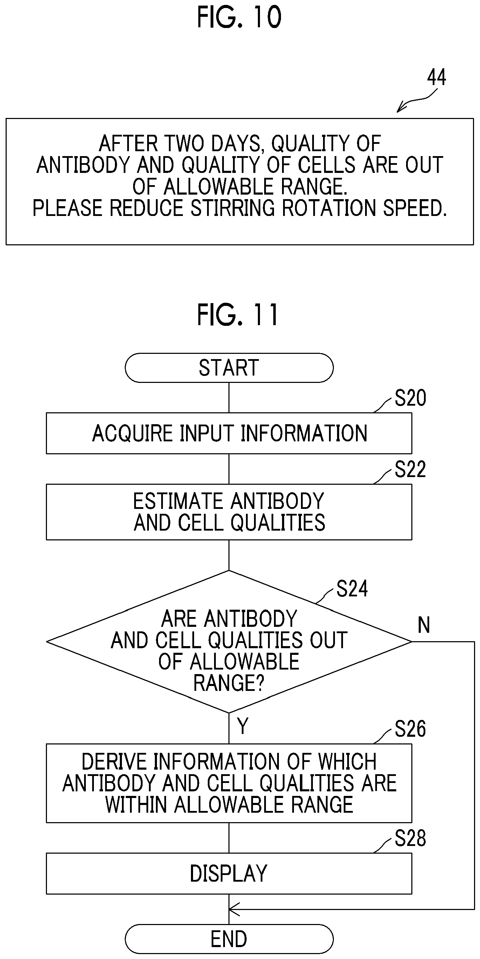

In a case where the determination unit 74 determines that the quality of the antibody and the quality of the cells are out of the allowable range, the output unit 78 outputs a warning to the display unit 44. Further, in such a case, the output unit 78 further outputs the information derived by the derivation unit 76 to the display unit 44. With the outputs, the warning screen shown in Fig. 10 is displayed on the display unit 44 as an example. As shown in Fig. 10, on the warning screen according to the present embodiment, a warning message indicating that the quality of the antibody and the quality of the cells are out of the allowable range is displayed after elapse of the predetermined period. Further, on the warning screen according to the present embodiment, explanatory variables in which the quality of the antibody and the quality of the cells are within the allowable range, and change information indicating how to change the explanatory variables are also displayed.

In a case where the explanatory variable derived by the derivation unit 76 is a parameter that can be controlled by the information processing apparatus 40, the output unit 78 may change the parameter by outputting the change information derived by the derivation unit 76 to the control target. Specifically, for example, in a case where the derivation unit 76 derives information indicating that the stirring rotation speed is reduced as change information, the output unit 78 outputs the change information to a motor that controls the stirring rotation speed. As a result, the stirring rotation speed is reduced by a predetermined rotation speed.

Next, referring to Fig. 11, an operation of the information processing apparatus 40 according to the present embodiment in the operation phase will be described. In a case where the CPU 41 executes the information processing program 52, quality estimation processing shown in Fig. 11 is executed. The quality estimation processing shown in Fig. 11 is executed at a periodic timing such as once a day after start of the perfusion culture.

In step S20 of Fig. 11, the acquisition unit 70 acquires the input information which is measured by the measurement unit 48. In step S22, as described above, the estimation unit 72 estimates the quality of the antibody and the quality of the cells after the predetermined period has elapsed from the time point at which the acquisition unit 70 acquires the input information by inputting the input information acquired in step S20 to the trained model 56.

In step S24, the determination unit 74 determines whether the quality of the antibody and the quality of the cells estimated in step S22 are out of the allowable range. In a case where the determination is affirmative, the processing proceeds to step S26. In step S26, as described above, the derivation unit 76 derives the explanatory variable having the highest level of contribution as the information included in the input information in which the quality of the antibody and the quality of the cells are within the allowable range. Further, as described above, the derivation unit 76 also derives change information indicating how the derived explanatory variable having the highest level of contribution is changed in accordance with whether the sign of the level of contribution is positive or negative. The trained model 56 may be retrained in accordance with the number of batches. In such a case, the weights between the nodes of the trained model 56 are also updated. Therefore, in the present embodiment, the processing of step S26 is executed every time the quality estimation processing is executed.

In step S28, the output unit 78 outputs the warning to the display unit 44 and outputs the information derived in step S26 to the display unit 44, as described above. Through the processing of step S28, the warning screen shown in Fig. 10 is displayed on the display unit 44 as an example. A user confirms the warning screen displayed on the display unit 44 through the processing of step S28, and adjusts the parameters such as the process conditions and the culture medium components that affect the quality of the antibody and the quality of the cells. In a case where the processing of step S28 is completed, the quality estimation processing is completed. Further, even in a case where the determination in step S24 is a negative determination, the quality estimation processing ends.

As described above, according to the present embodiment, the quality of the antibody and the quality of the cells are estimated after the predetermined period has elapsed from the time point at which the acquisition unit 70 acquires the input information. Therefore, the user is able to grasp the quality of the antibody and the quality of the cells after a predetermined period, and is able to adjust the parameters that affect the quality of the antibody and the quality of the cells in the perfusion culture. Therefore, it is possible to effectively support perfusion culture.

[Second Embodiment]

A second embodiment of the disclosed technique will be described. Since the configuration of the cell culture device 100 according to the present embodiment is similar to that of the first embodiment, the description thereof will not be repeated. The hardware configuration of the information processing apparatus 40 according to the present embodiment is similar to that of the first embodiment except for the learning data 54 and the trained model 56 stored in the storage unit 43. Therefore, the learning data 54 and the trained model 56 will be described.

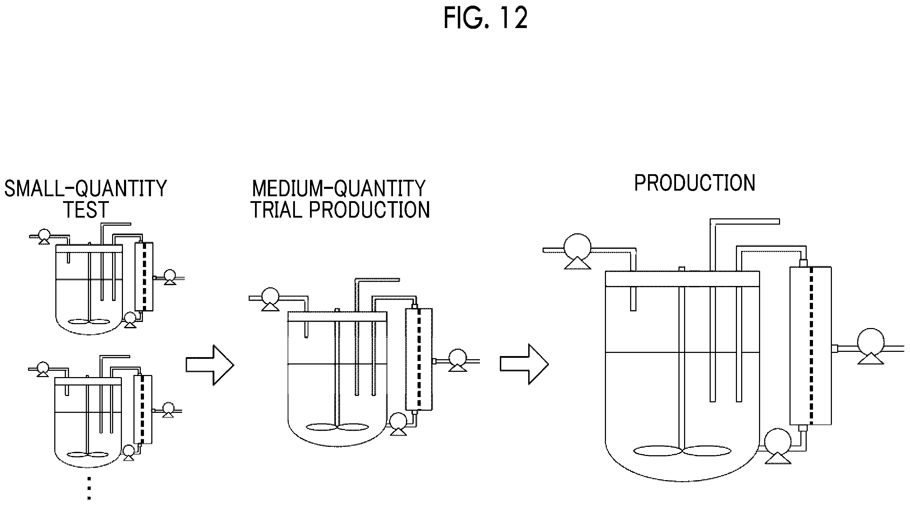

In the contract development of antibody drugs, cells are contracted from customers and antibodies are produced by culturing the contracted cells. In the contract development, as shown in Fig. 12, as an example, the optimum culture conditions are determined by a small-quantity test in which perfusion culture is performed under various process conditions and culture conditions such as culture medium components on a relatively small scale. Next, the qualities of the antibody and cells are confirmed by a medium-quantity trial production in which perfusion culture is performed under the culture conditions determined by the small-quantity test on a relatively medium scale. After confirmation of the qualities of the antibody and cells in the medium-quantity trial production is completed, the antibody is produced by perfusion culture on a relatively large scale.

The small-quantity test is performed, for example, for 30 days. In order to effectively support the perfusion culture, it is preferable that the period of the small-quantity test can be shortened.

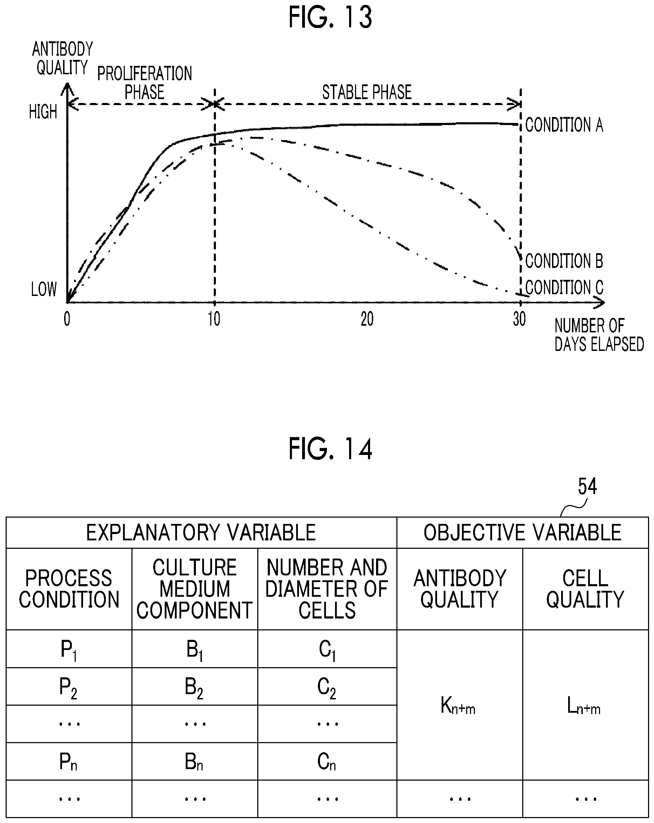

As an example, as shown in Fig. 13, the quality of the antibody improves with the passage of time in the cell proliferation phase such as 10 days after the start of perfusion culture. However, during the stable phase after the cell proliferation phase, the quality of the antibody may be stabilized (the conditions indicated by the solid line in the example of Fig. 13) or decreased (the conditions indicated by the one-dot chain line and the two-dot chain line in the example of Fig. 13) depending on the process conditions in the perfusion culture and the culture conditions such as the culture medium components. That is, the final quality of the antibody, such as after 30 days from the start of perfusion culture, varies depending on the conditions. Therefore, in the information processing apparatus 40 according to the present embodiment, the period of the small-quantity test is shortened by estimating the final quality of the antibody and the final quality of the cells from the measurement data at a plurality of time points in the cell proliferation phase.

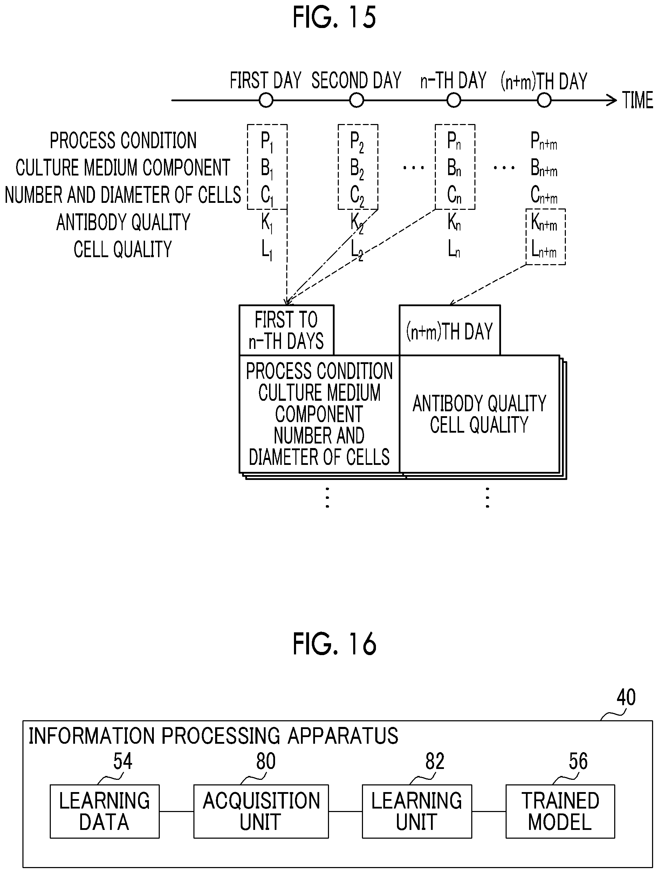

Referring to Figs. 14 and 15, the details of the learning data 54 according to the present embodiment will be described. As shown in Fig. 14, the learning data 54 is a data set for learning that includes a plurality of sets of process conditions, culture medium components, the number and diameters of cells in cell culture which are explanatory variables, a quality of the antibody produced from the cells which are objective variables corresponding to the explanatory variables, and a quality of the cells.

As shown in Fig. 15, in the present embodiment, the learning data 54 includes the process conditions, the culture medium components, and the number and diameters of cells acquired at a plurality of time points (every day in the example of Fig. 15) until a predetermined period n as the period for cell proliferation has elapsed from the start of cell culture (that is, within the cell proliferation phase). Further, in the present embodiment, the learning data 54 also includes a quality of the antibody and a quality of the cells after a predetermined period m has elapsed from the final acquisition of the process conditions, the culture medium components, and the number and diameters of cells associated with the process conditions, the culture medium components, and the number and diameters of cells acquired at the plurality of time points. For example, in a case where the small-quantity test is performed for 30 days and the cell proliferation phase is 10 days, n in Figs. 14 and 15 is 10 and m is 20. It should be noted that n and m are not limited to the example. For example, n may be 5 and m may be 9. That is, the process conditions at a plurality of time points for 5 days from the start of cell culture, the culture medium components, and the number and diameters of cells may be associated with a quality of the antibody and a quality of the cells after elapse of 14 days from the start of cell culture.

Next, referring to Fig. 16, a functional configuration in the learning phase of the information processing apparatus 40 according to the present embodiment will be described. As shown in Fig. 16, the information processing apparatus 40 includes an acquisition unit 80 and a learning unit 82. In a case where the CPU 41 executes the learning program 50, the CPU 41 functions as the acquisition unit 80 and the learning unit 82.

The acquisition unit 80 acquires the learning data 54 from the storage unit 43. The learning unit 82 generates the trained model 56 by training the model using the learning data 54 acquired by the acquisition unit 80 as training data. Then, the learning unit 82 stores the generated trained model 56 in the storage unit 43.

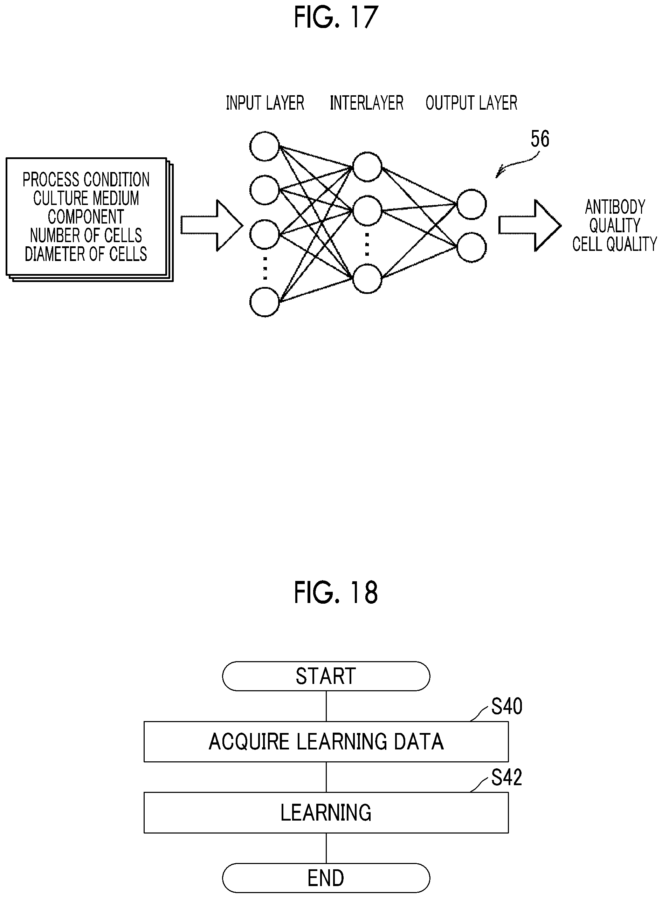

As an example, as shown in Fig. 17, by learning through the learning unit 82, the trained model 56 in which a plurality of sets of the process conditions, the culture medium components, the number and diameters of cells are input, and the quality of the antibody and the quality of cells are output are generated. For example, an error back propagation method is used in learning performed by the learning unit 82. The trained model 56 may be a deep neural network model having a plurality of interlayers. Further, as the trained model 56, a model other than the neural network may be applied.

Next, referring to Fig. 18, an operation of the information processing apparatus 40 according to the present embodiment in the learning phase will be described. In a case where the CPU 41 executes the learning program 50, the learning processing shown in Fig. 18 is executed.

In step S40 of Fig. 18, the acquisition unit 80 acquires the learning data 54 from the storage unit 43. In step S42, as described above, the learning unit 82 generates the trained model 56 by training the model using the learning data 54 acquired in step S40 as the training data. Then, the learning unit 82 stores the generated trained model 56 in the storage unit 43. In a case where step S42 ends, the learning processing ends.

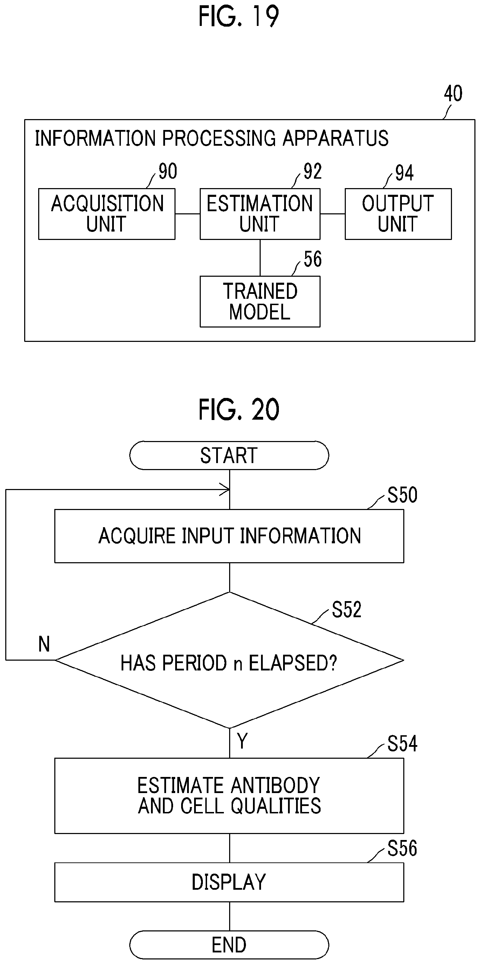

Next, referring to Fig. 19, a functional configuration in an operation phase of the information processing apparatus 40 will be described. As shown in Fig. 19, the information processing apparatus 40 includes an acquisition unit 90, an estimation unit 92, and an output unit 94. In a case where the CPU 41 executes the information processing program 52, the CPU 41 functions as an acquisition unit 90, an estimation unit 92, and an output unit 94.

The acquisition unit 90 acquires a plurality of sets of the process conditions, the culture medium components, and the number and diameters of cells in the cell culture using the cell culture device 100 measured by the measurement unit 48 at a plurality of time points until the predetermined period n as the period for cell proliferation has elapsed from the start of cell culture. The process condition, the culture medium component, and the number and diameters of cells are examples of input information input to the trained model 56.

The estimation unit 92 estimates the quality of the antibody and the quality of the cells after the predetermined period m has elapsed from the time point at which the acquisition unit 90 acquires the input information for the last time, on the basis of the plurality of sets of the input information acquired by the acquisition unit 90 at a plurality of time points and the trained model 56. Specifically, the estimation unit 92 inputs the plurality of sets of the input information acquired by the acquisition unit 90 to the trained model 56. As described above, the trained model 56 is a model that is trained in a case of inputting the plurality of sets of the process conditions, the culture medium components, the number and diameters of cells and outputting the quality of the antibody and the quality of the cells after elapse of the predetermined period m. Therefore, the output from the trained model 56 is estimated values of the quality of the antibody and the quality of the cells after the predetermined period m has elapsed from the time point at which the acquisition unit 90 acquires the input information for the last time. As described above, the estimation unit 92 estimates the quality of the antibody and the quality of the cells after the predetermined period m has elapsed from the time point at which the acquisition unit 90 acquires the input information for the last time.

The output unit 94 displays the quality of the antibody and the quality of the cells estimated by the estimation unit 92 by outputting the qualities to the display unit 44. Thereby, a user is able to know the final quality of the antibody and the final quality of the cells at a time point of the end of the cell proliferation phase. Therefore, the user is able to select a condition in which the quality of the antibody and the quality of the cells are expected to be the highest among the various conditions in the small-quantity test at an early stage, and perform the culture medium-quantity trial production.

Next, referring to Fig. 20, an operation of the information processing apparatus 40 according to the present embodiment in the operation phase will be described. In a case where the CPU 41 executes the information processing program 52, quality estimation processing shown in Fig. 20 is executed. The quality estimation processing shown in Fig. 20 is performed at the start of perfusion culture.

In step S50 of Fig. 20, the acquisition unit 90 acquires the input information which is measured by the measurement unit 48. In step S52, the acquisition unit 90 determines whether or not the predetermined period n as the period for cell proliferation has elapsed after start of the quality estimation processing (that is, after start of the cell culture). In a case where the determination is negative, the processing returns to step S50, and in a case where the determination is affirmative, the processing proceeds to step S54. Therefore, by repeatedly executing step S50 periodically until the predetermined period n as the period for cell proliferation has elapsed, the acquisition unit 90 acquires a plurality of sets of input information at a plurality of time points.

In step S54, as described above, the estimation unit 92 estimates the quality of the antibody and the quality of the cells after the predetermined period m has elapsed from the time point at which the acquisition unit 90 acquires the input information for the last time, on the basis of the plurality of sets of the input information acquired in step S50 at a plurality of time points and the trained model 56. In step S56, the output unit 94 displays the quality of the antibody and the quality of the cells estimated in step S54 by outputting to the display unit 44. In a case where the processing of step S56 is completed, the quality estimation processing is completed. In the second embodiment, the information processing apparatus 40 may monitor whether or not the quality of the antibody and the quality of the cells are within allowable range or adjust parameters, by using the trained model 56 according to the first embodiment, in a similar manner to the first embodiment, in the period after the cell proliferation phase (for example, the stable phase of Fig. 13).

As described above, according to the present embodiment, the quality of the antibody and the quality of the cells are estimated after the predetermined period m has elapsed from the time point at which the input information was acquired for the last time, on the basis of the plurality of input information pieces acquired at the plurality of time points until the predetermined period n as the period for cell proliferation has elapsed from the start of cell culture. Therefore, as a result of shortening the period of the small-quantity test, it is possible to effectively support perfusion culture.

As the hardware structure of the processing unit that executes various processes such as each functional unit of the information processing apparatus 40 in each of the above-mentioned embodiments, it is possible to use various processors to be described below. As described above, various processors include not only a CPU as a general-purpose processor which functions as various processing units by executing software (programs) but also a programmable logic device (PLD) as a processor capable of changing a circuit configuration after manufacturing a field programmable gate array (FPGA); and a dedicated electrical circuit as a processor, which has a circuit configuration specifically designed to execute specific processing, such as an application specific integrated circuit (ASIC).

One processing unit may be configured as one of the various processors, or may be configured as a combination of two or more of the same or different kinds of processors (for example, a combination of a plurality of FPGAs or a combination of a CPU and an FPGA). Further, the plurality of processing units may be composed of one processor.

As an example of the plurality of processing units composed of one processor, first, as represented by computers such as a client and a server, there is a form in which one processor is composed of a combination of one or more CPUs and software and this processor functions as a plurality of processing units. Second, as represented by a system on chip (SoC), there is a form in which a processor that realizes the functions of the whole system including a plurality of processing units with a single integrated circuit (IC) chip is used. As described above, the various processing units are configured by using one or more of the various processors as a hardware structure.

Furthermore, as the hardware structure of these various processors, more specifically, it is possible to use an electric circuit (circuitry) in which circuit elements such as semiconductor elements are combined.

Further, in each of the above-mentioned embodiments, the configuration in which the learning program 50 and the information processing program 52 are stored (installed) in the storage unit 43 in advance has been described, but the present invention is not limited thereto. The learning program 50 and the information processing program 52 may be provided in a form in which the programs are stored in a storage medium such as a compact disc read only memory (CD-ROM), a digital versatile disc read only memory (DVD-ROM), and a universal serial bus (USB) memory. Further, the learning program 50 and the information processing program 52 may be downloaded from an external device through a network.

From the above-mentioned description, the technology relating to the following supplementary items can be found.

[Additional Notes]

An information processing apparatus comprising:

a processor; and
a memory that is built into or connected to the processor,
in which the processor is configured to

acquire input information including at least one of a process condition, a culture medium component, or the number and diameters of cells in cell culture, and
estimate a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

The present disclosure of JP2019-173364 filed on September 24, 2019 is incorporated herein by reference in its entirety. Further, all documents, patent applications, and technical standards described in the present specification are incorporated into the present specification by reference to the same extent as in a case where the individual documents, patent applications, and technical standards were specifically and individually stated to be incorporated by reference.

**Claims**

1. An information processing apparatus comprising:

an acquisition unit that acquires input information including at least one of a process condition, a culture medium component, or the number and diameters of cells in cell culture; and
an estimation unit that estimates a quality of an antibody produced from the cells and a quality of the cells after elapse of a predetermined period from a time point at which the acquisition unit acquires the input information, on the basis of the input information and a trained model which is trained in advance using the input information, the quality of the antibody, and the quality of the cells.

2. The information processing apparatus according to claim 1, further comprising an output unit that outputs a warning in a case where the quality of the antibody and the quality of the cells estimated by the estimation unit are out of an allowable range.

3. The information processing apparatus according to claim 2, wherein the output unit further outputs at least one information included in the input information in which the quality of the antibody and the quality of the cells are within the allowable range.

4. The information processing apparatus according to any one of claims 1 to 3, wherein an input of the trained model is a plurality of pieces of the input information acquired at a plurality of time points until the predetermined period as a period for cell proliferation has elapsed from start of the cell culture.

5. An information processing method executed by a computer, the method comprising:

   acquiring input information including at least one of a process condition, a culture medium component, or the number and diameters of cells in cell culture; and
   estimating a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

6. An information processing program for causing a computer to execute:

   acquiring input information including at least one of a process condition, a culture medium component, or the number and diameters of cells in cell culture; and
   estimating a quality of an antibody produced from the cells and a quality of the cells after elapse of a predetermined period from a time point at which the input information is acquired, on the basis of the input information and a trained model which is trained in advance using the input information, the quality of the antibody, and the quality of the cells.

FIG. 1

EP 4 015 616 A1

# FIG. 2

40

CPU 41

MEMORY 42

DISPLAY UNIT 44

47

STORAGE UNIT

50 — LEARNING PROGRAM

52 — INFORMATION PROCESSING PROGRAM

54 — LEARNING DATA

56 — TRAINED MODEL

43

INPUT UNIT

45

EXTERNAL I/F — 46

MEASUREMENT UNIT — 48

# FIG. 3

54

| EXPLANATORY VARIABLE | | | OBJECTIVE VARIABLE | |
|---|---|---|---|---|
| PROCESS CONDITION | CULTURE MEDIUM COMPONENT | NUMBER AND DIAMETER OF CELLS | ANTIBODY QUALITY | CELL QUALITY |
| $P_1$ | $B_1$ | $C_1$ | $K_3$ | $L_3$ |
| $P_2$ | $B_2$ | $C_2$ | $K_4$ | $L_4$ |
| ... | ... | ... | ... | ... |
| $P_n$ | $B_n$ | $C_n$ | $K_{n+2}$ | $L_{n+2}$ |
| ... | ... | ... | ... | ... |

# FIG. 4

FIRST DAY    SECOND DAY    THIRD DAY   FOURTH DAY

⟶ TIME

PROCESS CONDITION   $P_1$   $P_2$   $P_3$   $P_4$

CULTURE MEDIUM COMPONENT   $B_1$   $B_2$   $B_3$   $B_4$   $\cdots$

NUMBER AND DIAMETER OF CELLS   $C_1$   $C_2$   $C_3$   $C_4$

ANTIBODY QUALITY   $K_1$   $K_2$   $K_3$   $K_4$

CELL QUALITY   $L_1$   $L_2$   $L_3$   $L_4$

| $n$-TH DAY | $(n+2)$TH DAY |
|---|---|
| PROCESS CONDITION CULTURE MEDIUM COMPONENT NUMBER AND DIAMETER OF CELLS | ANTIBODY QUALITY CELL QUALITY |

# FIG. 5

40

**INFORMATION PROCESSING APPARATUS**

| 54 | 60 | 62 | 56 |
|---|---|---|---|
| LEARNING DATA | ACQUISITION UNIT | LEARNING UNIT | TRAINED MODEL |

# FIG. 6

INPUT LAYER    INTERLAYER    OUTPUT LAYER

56

PROCESS CONDITION
CULTURE MEDIUM COMPONENT
NUMBER OF CELLS
DIAMETER OF CELLS

⟹

⟹ ANTIBODY QUALITY
CELL QUALITY

## FIG. 7

```
        START
          |
ACQUIRE LEARNING DATA      S10
          |
       LEARNING            S12
          |
         END
```

## FIG. 8

40

INFORMATION PROCESSING APPARATUS

| ACQUISITION UNIT (70) | ESTIMATION UNIT (72) | DETERMINATION UNIT (74) | DERIVATION UNIT (76) | OUTPUT UNIT (78) |

TRAINED MODEL (56)

## FIG. 9

INPUT LAYER    INTERLAYER    OUTPUT LAYER

$W_{1A}$
$W_{1B}$
$W_{AO}$
$W_{BO}$

# FIG. 10

44

AFTER TWO DAYS, QUALITY OF
ANTIBODY AND QUALITY OF CELLS ARE OUT
OF ALLOWABLE RANGE.
PLEASE REDUCE STIRRING ROTATION SPEED.

# FIG. 11

START

ACQUIRE INPUT INFORMATION — S20

ESTIMATE ANTIBODY
AND CELL QUALITIES — S22

ARE ANTIBODY
AND CELL QUALITIES OUT
OF ALLOWABLE
RANGE? — S24

N

Y

DERIVE INFORMATION OF WHICH
ANTIBODY AND CELL QUALITIES ARE
WITHIN ALLOWABLE RANGE — S26

DISPLAY — S28

END

# FIG. 12

SMALL-QUANTITY TEST

MEDIUM-QUANTITY TRIAL PRODUCTION

PRODUCTION

EP 4 015 616 A1

## FIG. 13

## FIG. 14

54

| EXPLANATORY VARIABLE | | | OBJECTIVE VARIABLE | |
|---|---|---|---|---|
| PROCESS CONDITION | CULTURE MEDIUM COMPONENT | NUMBER AND DIAMETER OF CELLS | ANTIBODY QUALITY | CELL QUALITY |
| $P_1$ | $B_1$ | $C_1$ | | |
| $P_2$ | $B_2$ | $C_2$ | $K_{n+m}$ | $L_{n+m}$ |
| . . . | . . . | . . . | | |
| $P_n$ | $B_n$ | $C_n$ | | |
| . . . | . . . | . . . | . . . | . . . |

# FIG. 15

FIRST DAY   SECOND DAY    n-TH DAY    (n+m)TH DAY

⟶ TIME

PROCESS CONDITION $\qquad$ $P_1$ $\qquad$ $P_2$ $\qquad$ $P_n$ $\qquad$ $P_{n+m}$
CULTURE MEDIUM COMPONENT $\qquad$ $B_1$ $\qquad$ $B_2$ $\cdots$ $B_n$ $\cdots$ $B_{n+m}$
NUMBER AND DIAMETER OF CELLS $\qquad$ $C_1$ $\qquad$ $C_2$ $\qquad$ $C_n$ $\qquad$ $C_{n+m}$
ANTIBODY QUALITY $\qquad$ $K_1$ $\qquad$ $K_2$ $\qquad$ $K_n$ $\qquad$ $K_{n+m}$
CELL QUALITY $\qquad$ $L_1$ $\qquad$ $L_2$ $\qquad$ $L_n$ $\qquad$ $L_{n+m}$

FIRST TO n-TH DAYS

PROCESS CONDITION
CULTURE MEDIUM COMPONENT
NUMBER AND DIAMETER OF CELLS

(n+m)TH DAY

ANTIBODY QUALITY
CELL QUALITY

# FIG. 16

40

INFORMATION PROCESSING APPARATUS

| 54 | 80 | 82 | 56 |
|---|---|---|---|
| LEARNING DATA | ACQUISITION UNIT | LEARNING UNIT | TRAINED MODEL |

## FIG. 17

INPUT LAYER    INTERLAYER    OUTPUT LAYER

56

PROCESS CONDITION
CULTURE MEDIUM
COMPONENT
NUMBER OF CELLS
DIAMETER OF CELLS

ANTIBODY
QUALITY
CELL QUALITY

## FIG. 18

START

ACQUIRE LEARNING DATA   S40

LEARNING   S42

END

## FIG. 19

40

INFORMATION PROCESSING APPARATUS

```
        90              92              94
ACQUISITION     ESTIMATION        OUTPUT
   UNIT            UNIT            UNIT
                    56
                 TRAINED
                  MODEL
```

## FIG. 20

START

ACQUIRE INPUT INFORMATION ⟍S50

HAS PERIOD n ELAPSED? ⟍S52

N

Y

ESTIMATE ANTIBODY AND CELL QUALITIES ⟍S54

DISPLAY ⟍S56

END

## INTERNATIONAL SEARCH REPORT

| International application No. |
|---|
| PCT/JP2020/018808 |

| A. CLASSIFICATION OF SUBJECT MATTER |
|---|
| C12M 1/00(2006.01)i; C12M 1/34(2006.01)i; G16B 40/00(2019.01)i<br>FI: C12M1/00 C; G16B40/00; C12M1/34 A<br>According to International Patent Classification (IPC) or to both national classification and IPC |

| B. FIELDS SEARCHED |
|---|
| Minimum documentation searched (classification system followed by classification symbols)<br>C12M1/00; C12M1/34; G16B40/00 |

| Documentation searched other than minimum documentation to the extent that such documents are included in the fields searched |  |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

| Electronic data base consulted during the international search (name of data base and, where practicable, search terms used) |
|---|
| JSTPlus/JMEDPlus/JST7580 (JDreamIII) |

| C. DOCUMENTS CONSIDERED TO BE RELEVANT |

| Category* | Citation of document, with indication, where appropriate, of the relevant passages | Relevant to claim No. |
|---|---|---|
| A | JP 2009-44974 A (NAGOYA UNIVERSITY) 05.03.2009 (2009-03-05) entire text | 1-6 |
| A | JP 2018-117567 A (HITACHI, LTD.) 02.08.2018 (2018-08-02) entire text | 1-6 |
| A | WO 2018/142702 A1 (NIKON CORP.) 09.08.2018 (2018-08-09) entire text | 1-6 |
| P, A | WO 2019/181234 A1 (FUJIFILM CORPORATION) 26.09.2019 (2019-09-26) entire text | 1-6 |
| P, A | WO 2020/039683 A1 (FUJIFILM CORPORATION) 27.02.2020 (2020-02-27) entire text | 1-6 |
| P, A | WO 2020/021860 A1 (FUJIFILM CORPORATION) 30.01.2020 (2020-01-30) entire text | 1-6 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 July 2020 (10.07.2020) | 21 July 2020 (21.07.2020) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**
Information on patent family members

| | | International application no. |
|---|---|---|
| | | PCT/JP2020/018808 |

| Patent Documents referred in the Report | Publication Date | Patent Family | Publication Date |
|---|---|---|---|
| JP 2009-44974 A | 05 Mar. 2009 | (Family: none) | |
| JP 2018-117567 A | 02 Aug. 2018 | (Family: none) | |
| WO 2018/142702 A1 | 09 Aug. 2018 | US 2019/0347798 A1 EP 3578633 A1 | |
| WO 2019/181234 A1 | 26 Sep. 2019 | (Family: none) | |
| WO 2020/039683 A1 | 27 Feb. 2020 | (Family: none) | |
| WO 2020/021860 A1 | 30 Jan. 2020 | (Family: none) | |

Form PCT/ISA/210 (patent family annex) (January 2015)

**REFERENCES CITED IN THE DESCRIPTION**

**Patent documents cited in the description**

- JP 2009044974 A

- JP 2019173364 A